# DEMANDE DE BREVET EUROPEEN

(11) **EP 4 530 626 A1**
(43) Date de publication de la demande: **02.04.2025**
(21) Numéro de dépôt: 24199547.1
(22) Date de dépôt: 10.09.2024
(51) Int. Cl.: G01N 33/14, G01N 33/94

(54) **PROCEDE DE DETECTION D'INTRODUCTION DE GAMMA-HYDROXYBUTYRATE DANS UNE SOLUTION**

(30) Priorité: 28.09.2023 FR 2310370
(71) Demandeur: Commissariat à l'Energie Atomique et aux Energies Alternatives, 75015 Paris (FR)
(72) Inventeur: SCORSONE, Emmanuel, 91191 GIF-SUR-YVETTE CEDEX (FR)
(74) Mandataire: Atout PI Laplace

(57) **Abrégé**

L'invention se rapporte à un Procédé de détection (P) d'introduction de gamma-hydroxybutyrate dans une solution (Sol) au moyen d'un capteur (Capt), le capteur comprenant une électrode de travail (Ew), une électrode de référence (Eref), une contre-électrode (Ec) et un circuit potentiostatique (CP) connecté à l'électrode de travail (Ew), l'électrode de référence (Eref) et la contre-électrode (Ec), le procédé comprenant les étapes suivantes :
- alterner, au moyen du circuit potentiostatique et pendant une durée prédéterminée, une tension de polarisation de l'électrode de travail entre une tension minimum et une tension maximum prédéterminées, la tension minimum étant choisie dans une gamme de potentiels produisant un courant de réduction du gamma-hydroxybutyrate, la tension maximum étant choisie à l'extérieur de ladite gamme de potentiels ;
- mesurer, au moyen du circuit potentiostatique, un courant au niveau de l'électrode de travail pendant ladite durée prédéterminée ; et
- analyser le courant mesuré, une détection d'une variation du courant entre un premier instant et un deuxième instant ultérieur au premier instant au cours de ladite étant caractéristique de l'introduction de gamma-hydroxybutyrate.

## Description

### DOMAINE TECHNIQUE

L'invention concerne un procédé de détection d'introduction de gamma-hydroxybutyrate dans une solution et en particulier un procédé de détection utilisant un capteur chimique utilisant une méthode électrochimique permettant de produire un courant électrique spécifique lors de l'introduction de gamma-hydroxybutyrate dans une solution. De plus, l'invention concerne un capteur permettant la détection d'introduction de gamma-hydroxybutyrate dans une solution et en particulier un capteur chimique. De plus, l'invention concerne un contenant comprenant un capteur chimique et permettant la détection d'introduction de gamma-hydroxybutyrate dans une solution.

### ARRIÈRE-PLAN

Il n'existe à ce jour aucun dispositif permettant la détection de l'introduction du gamma-hydroxybutyrate (GHB) dans un verre de boisson en temps réel. La plupart des méthodes de détection rapide du GHB sont à transduction optique. Il s'agit de sondes chimiques fluorescentes ou colorimétriques. Parmi les quelques sondes fluorescentes, il existe le « GHB Orange » [Duanting Zhai, Yong Qiao Elton Tan, Wang Xu and Young-Tae Chang, Development of a fluorescent sensor for illicit date rape drug GHB Chem. Commun., 2014,50, 2904-2906], dans lequel l'approche consiste à prélever un échantillon de boisson et à le verser dans une solution contenant la sonde fluorescente et de préférence 50% de solvant diméthylsulfoxyde. En présence de GHB, un signal fluorescent est observé sous irradiation à l'aide d'une lampe à rayons ultraviolets. Une autre approche similaire consiste à utiliser un réseau de sondes fluorescentes immobilisées dans des capsules organiques (cucurbiturils). Chaque paire sonde/capsule est disposée dans un puit. Après le prélèvement d'un échantillon de boisson et enlèvement de l'alcool présent (qui constitue un interférent pour l'analyse), l'échantillon est réparti dans les différents puits pour produire une émission de fluorescence variable en fonction de la boisson. En présence de GHB une « empreinte colorimétrique » spécifique peut être observée [Baumes, L., BuakiSogo, M., Montes-Navajas, P., Corma, A. and Garcia, H. (2010), A Colorimetric Sensor Array for the Detection of the Date-Rape Drug γ-Hydroxybutyric Acid (GHB): A Supramolecular Approach. Chemistry - A European Journal, 16: 4489-4495.]. D'autres sondes colorimétriques ont également été utilisées qui sont à l'origine de tentative de produits commerciaux (DrinkSafe^{™} cards, DrinkSafe^{™} coasters, Drink Detective^{™}" de chez Drink Safe Technology^{®}, ou EZ Test kit pour GHB de chez EZ Test Kits^{®}). Le principe est toujours le même, il consiste à mettre le liquide (la boisson) en contact avec la sonde pour produire un changement de couleur irréversible. De telles sondes colorimétriques peuvent être immobilisées dans des pailles en plastique, substrats en papier, et même vernis à ongles. Toutes ces méthodes souffrent d'un manque de fiabilité (taux élevé de faux positifs), de non réversibilité (usage unique), ils nécessitent une action, plus ou moins contraignante du consommateur (prélèvement). Elles ne sont pas compatibles avec une intégration dans un verre pour une lecture en temps réel.

Une autre approche proposée consiste à procéder à une analyse électrochimique. Cette approche a été proposée par R. Jiménez-Pérez et ses collaborateurs à partir d'électrodes à base de platine [R. Jiménez-Pérez, J.M. Sevilla, T. Pineda, M. Blázquez, J. Gonzalez-Rodriguez, Electrocatalytic performance enhanced of the electrooxidation of gamma-hydroxybutyric acid (GHB) and ethanol on platinum nanoparticles surface. A contribution to the analytical détermination of GHB in the presence of ethanol, Sensors and Actuators B: Chemical, Volume 256, 2018, Pages 553-563, R. Jiménez-Pérez, J.M. Sevilla, T. Pineda, M. Blázquez, J. Gonzalez-Rodriguez, Study of the electro-oxidation of a recreational drug GHB (gamma hydroxybutyric acid) on a platinum catalyst-type electrode through chronoamperometry and spectro-electrochemistry, Journal of Electroanalytical Chemistry, Volume 766, 2016, Pages 141-146, R. Jiménez-Pérez, J.M. Sevilla, T. Pineda, M. Blázquez, J. Gonzâlez-Rodriguez, Electrochemical behaviour of gamma hydroxybutyric acid at a platinum electrode in acidic medium, Electrochimica Acta, Volume 111, 2013, Pages 601-607]. La méthode repose sur des mesures par voltammétrie cyclique. Elle consiste à mesurer l'oxydation de l'alcool (éthanol) dans le verre. L'adsorption du GHB et de l'éthanol sur la surface de l'électrode sont compétitives et est plutôt favorable au GHB. L'oxydation du GHB étant moins prononcée que celle de l'éthanol sur une électrode platine, la mesure consiste alors à utiliser la baisse du signal d'oxydation de l'éthanol observé par l'ajout de GHB dans le verre lorsque le GHB se fixe sur l'électrode quand un potentiel cathodique est appliqué. Cette méthode présente des inconvénients : le premier est que la boisson doit comporter une quantité d'alcool non négligeable. Elle ne s'applique donc qu'aux boissons alcoolisées. La seconde et sans doute la plus importante, est que la mesure ne peut se faire qu'en mode statique, c'est-à-dire sans agitation du verre. La réponse ampérométrique est très dépendante de la diffusion des espèces à proximité de l'électrode. Cette diffusion est très impactée par l'agitation du liquide. La mesure n'est donc potentiellement fiable que si le verre est immobile pendant la durée de l'analyse (plusieurs dizaines de secondes). Elle peut donc difficilement s'appliquer sur le terrain (milieux festifs, etc.) quand le consommateur porte son verre à la main ou le prend fréquemment en main pour en boire le contenu.

### RÉSUMÉ

Afin de pallier les inconvénients des procédés de détection d'introduction de gamma-hydroxybutyrate dans une solution existants, l'objectif de la présente invention est de proposer un procédé de détection réversible de l'introduction de GHB dans une solution (telle qu'une boisson). En particulier, le procédé est mis en oeuvre par un capteur qui peut être placé dans un contenant (tel qu'un verre dans lequel la boisson est placée). Le capteur doit être suffisamment sensible pour provoquer la détection. Il doit également être sélectif, en particulier afin de ne pas provoquer de faux positif qui induiraient une perte de confiance envers le capteur de la part de l'utilisateur. L'invention est utilisable pour une variété de boissons possibles, qui chacune peuvent comporter un mélange complexe de molécules différentes, dans des conditions de pH différents. En d'autres termes, l'invention permet d'être sélectif à une molécule dans un milieu complexe et variable. Le capteur doit également être réversible, afin de permettre sa réutilisation (et donc celle du contenant) lorsque le capteur est intégré au contenant. De plus, le matériau sensible, en contact avec la boisson, doit être composé d'un matériau non toxique, compatible avec les normes d'hygiène et de sécurité alimentaire et résistant aux conditions habituelles d'utilisation (telles que le lavage avec un liquide vaisselle ou par un lave-vaisselle). Le capteur est également relativement insensible aux mouvements, ou au volume de liquide dans le verre. Enfin, le capteur est peu consommateur en énergie pour pouvoir être alimenté par batterie (de préférence pile bouton facilement intégrable au fond du verre) le temps d'un évènement (soirée, mariage, etc.), c'est-à-dire typiquement pour une durée minimale d'environ 12h. En effet, le capteur selon l'invention est un capteur ampérométrique. Lorsqu'une tension est appliquée, un courant est mesuré, ce dernier étant produit par l'oxydation ou la réduction des espèces chimiques présentes dans la solution. En dehors de l'alimentation du circuit électronique, aucun composant consommateur d'énergie n'est donc requis, permettant ainsi d'alimenter le capteur par batterie.

A cet effet, l'invention a pour premier objet un procédé de détection d'introduction de gamma-hydroxybutyrate dans une solution au moyen d'un capteur, le capteur comprenant une électrode de travail, une électrode de référence, une contre-électrode et un circuit potentiostatique connecté à l'électrode de travail, l'électrode de référence et la contre-électrode, le procédé comprenant les étapes suivantes :
- alterner, au moyen du circuit potentiostatique et pendant une durée prédéterminée, une tension de polarisation de l'électrode de travail entre une tension minimum et une tension maximum prédéterminées, la tension minimum étant choisie dans une gamme de potentiels produisant un courant de réduction du gamma-hydroxybutyrate, la tension maximum étant choisie à l'extérieur de ladite gamme de potentiels ;
- mesurer, au moyen du circuit potentiostatique, un courant au niveau de l'électrode de travail pendant ladite durée prédéterminée ; et
- analyser le courant mesuré, une détection d'une variation du courant entre un premier instant et un deuxième instant ultérieur au premier instant au cours de ladite analyse étant caractéristique de l'introduction de gamma-hydroxybutyrate.

Avantageusement, l'invention fait appel à des procédés électrochimiques relativement simples de mise en oeuvre, miniaturisables, peu consommateurs d'énergie et donc intégrables dans un contenant (par exemple un verre) avec une alimentation sur batterie de type « pile bouton ». L'élément sensible du capteur peut être composé d'un métal noble avec une bonne compatibilité alimentaire. Ce dernier est extrêmement robuste à une exposition à des boissons qui peuvent parfois être corrosives (le pH moyen de beaucoup de boissons tourne autour de pH=2 à pH=4). Il est également compatible avec un lavage manuel ou au lave-vaisselle, en présence de détergents puissants. Un autre avantage de l'invention est que le procédé de détection est réversible (en opposition par exemples aux dissipatifs optiques. La mesure est automatique et ne nécessite pas d'intervention particulière du consommateur (prélèvement d'échantillons etc., comme on peut le voir sur certains tests colorimétriques). Enfin, par rapport à d'autres types de détections électrochimiques qui pourraient se trouver dans la littérature, la méthode fonctionne à la fois lorsque le verre est en statique ou en mouvement. La combinaison d'un capteur électrochimique à la fois sensible au GHB, sélectif dans le cadre applicatif, réutilisable et fonctionnant dans un verre en mouvement, constitue l'innovation principale de cette invention. Il n'existe à notre connaissance aucun capteur sur le marché ou dans la littérature scientifique remplissant tous ces critères à la fois. L'étape d'alternance entre une tension minimale et une tension maximale de façon répétée dans le temps permet ainsi de détecter le GHB dans un verre en mouvement. De plus, cette alternance permet également de détecter le GHB dans une boisson ne contenant pas d'alcool.

Dans un mode de réalisation, l'introduction du gamma-hydroxybutyrate est détectée lorsque la variation du courant est supérieure ou égale à une valeur prédéfinie.

Avantageusement, la valeur prédéfinie permet d'éviter les faux positifs liés par exemple aux mouvements de l'utilisateur.

Dans un mode de réalisation, l'étape d'analyser comprend les sous-étapes suivantes :
- déterminer une valeur moyenne du courant correspondant à une différence entre une valeur moyenne du courant lorsque la tension de polarisation de l'électrode de travail est égale à la tension minimum et une valeur moyenne du courant lorsque la tension de polarisation de l'électrode de travail est égale à la tension maximum en fonction du temps ;
- déterminer une première différence entre la valeur moyenne du courant au premier instant et la valeur moyenne du courant au deuxième instant; et
- détecter l'introduction du gamma-hydroxybutyrate lorsque la valeur absolue de la première différence est supérieure à un premier seuil prédéterminé.

Avantageusement, ces sous-étapes d'analyse permettent de visualiser de façon précise les variations caractéristiques de l'introduction du GHB.

Dans un mode de réalisation, l'étape d'analyser comprend les sous-étapes suivantes :
- déterminer une valeur moyenne du courant lorsque la tension de polarisation de l'électrode de travail est égale à la tension maximum en fonction du temps ;
- détecter l'introduction du gamma-hydroxybutyrate lorsque la valeur absolue de la première différence est supérieure au premier seuil prédéterminé et la valeur moyenne du courant lorsque la tension de polarisation de l'électrode de travail égale à la tension maximum est environ constante entre le premier instant et le deuxième instant.

Avantageusement, ces sous-étapes d'analyse permettent de prendre en compte les mouvements du contenant.

Dans un mode de réalisation, l'étape d'analyse comprend les sous-étapes suivantes :
- déterminer une valeur moyenne du courant lorsque la tension de polarisation de l'électrode de travail est égale à la tension minimum en fonction du temp s ;
- déterminer une deuxième différence entre la valeur moyenne du courant au premier instant et la valeur moyenne du courant au deuxième instant temps ; et
- détecter l'introduction du gamma-hydroxybutyrate lorsque la valeur absolue de la deuxième différence est supérieure à un deuxième seuil prédéterminé.

Avantageusement, ces sous-étapes d'analyse permettent de visualiser de façon précise les variations caractéristiques de l'introduction du GHB même en cas de vibrations ou de mouvements du verre.

Dans un mode de réalisation, le procédé comprend :
- si le courant présente une variation, émettre un signal d'alerte.

Dans un mode de réalisation, le signal d'alerte est émis lorsque la variation est supérieure à la valeur prédéfinie pendant une deuxième durée prédéterminée.

Avantageusement, la deuxième durée prédéterminée permet d'éviter de faux positifs dus à un mouvement du verre ou une vibration.

Dans un mode de réalisation, le signal d'alerte est un signal sonore, lumineux ou vibrant.

Avantageusement, le signal d'alerte permet d'alerter l'utilisateur que du GHB a été introduit dans le verre.

Dans un mode de réalisation, la gamme de tension de l'électrode de travail est choisie entre -2.2 V/Pt et -1.6 V/Pt pour la tension minimum et -1.4 V/Pt et 0 V/Pt pour la tension maximum, l'électrode de référence étant en platine.

Avantageusement, cette gamme de tension permet d'assurer la réduction du GHB et ainsi sa détection tout en évitant la réduction de l'eau présente dans la solution et ainsi évitant un signal bruité.

L'invention a pour deuxième objet un capteur de détection d'introduction de gamma-hydroxybutyrate dans une solution, le capteur étant placé dans un contenant et étant en contact avec la solution, le capteur comprenant une électrode de travail, une électrode de référence et une contre électrode et un circuit potentiostatique connecté à l'électrode de travail, l'électrode de référence et la contre-électrode, le capteur étant configuré pour mettre en oeuvre le procédé selon le premier objet de l'invention.

Dans un mode de réalisation, l'électrode de travail étant en métal noble, de préférence en platine.

Avantageusement, le platine permet au capteur d'être résistant au lavage en lave-vaisselle, aux produits nettoyants pour la vaisselle, et aux pH variés des boissons.

Dans un mode de réalisation, l'électrode de travail a une surface comprise entre 0.002 mm² et 100 mm².

Avantageusement, cette surface permet de réduire les coûts associés aux électrodes.

L'invention a pour troisième objet un contenant permettant la détection d'une introduction de gamma-hydroxybutyrate dans un liquide placé dans le contenant, le contenant comprenant le capteur selon le deuxième objet de l'invention.

Dans un mode de réalisation, le contenant comprend un système d'alerte configuré pour émettre le signal d'alerte, le signal d'alerte étant de préférence un signal sonore, lumineux ou vibrant.

Avantageusement, le signal d'alerte permet d'alerter l'utilisateur que du GHB a été introduit dans verre.

### FIGURES

L'invention sera mieux comprise et d'autres avantages apparaîtront à la lecture de la description qui va suivre donnée à titre non limitatif et grâce aux figures, parmi lesquelles :
[Fig.1] la figure 1 illustre un procédé de détection d'introduction de gamma-hydroxybutyrate dans une solution selon l'invention ;
[Fig. 2a] la figure 2a illustre un exemple de courant mesuré selon l'invention ;
[Fig. 2b] la figure 2b illustre un exemple de courant mesuré selon l'invention ;
[Fig. 3a] la figure 3a illustre un exemple de courant mesuré selon l'invention;
[Fig. 3b] la figure 3b illustre un exemple d'analyse de courant mesuré selon l'invention ;
[Fig. 3c] la figure 3c illustre un exemple d'analyse de courant mesuré selon l'invention ;
[Fig. 3d] la figure 3d illustre un exemple d'analyse de courant mesuré selon l'invention ;
[Fig. 4a] la figure 4a illustre un exemple de courant mesuré selon l'invention;
[Fig. 4b] la figure 4b illustre un exemple d'analyse du courant mesuré selon l'invention ;
[Fig. 4c] la figure 4c illustre un exemple d'analyse du courant mesuré selon l'invention ;
[Fig. 4d] la figure 4d illustre un exemple d'analyse du courant mesuré selon l'invention ;
[Fig. 5] la figure 5 illustre un exemple de capteur selon l'invention ; et
[Fig. 6] la figure 6 illustre un exemple de contenant selon l'invention.

### DESCRIPTION DÉTAILLÉE

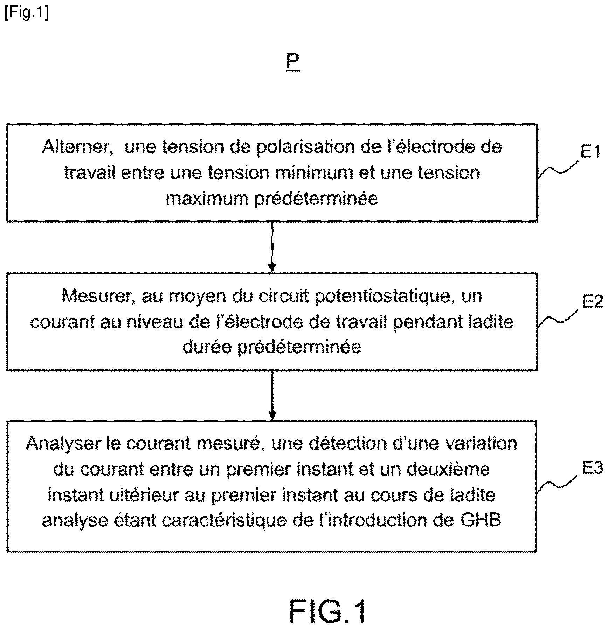

L'invention concerne un procédé de détection d'introduction de gamma-hydroxybutyrate dans une solution. La figure 1 illustre un procédé de détection P selon l'invention.

Le procédé de détection P d'introduction de gamma-hydroxybutyrate (GHB) dans une solution Sol est mis en oeuvre au moyen d'un capteur Capt, le capteur comprenant une électrode de travail Ew, une électrode de référence Eref, une contre-électrode Ec et un circuit potentiostatique CP connecté à l'électrode de travail Ew, à l'électrode de référence Eref et à la contre-électrode Ec.

Le gamma-hydroxybutyrate (GHB) peut être ajouté dans une solution (par exemple une boisson) sous forme solide (poudre, gélule) ou sous forme liquide lorsque celui-ci a préalablement été dissous par exemple dans un peu d'eau. La dose de GHB nécessaire à un acte de soumission chimique dépend de plusieurs facteurs : poids et sexe de la victime potentielle, victime sous emprise d'alcool ou pas, etc. Néanmoins, de manière générale, il est considéré que la dose moyenne à administrer à une personne pour entrer dans le domaine de la soumission chimique se situe autour de 1 gramme.

La solution Sol peut être par exemple un liquide tel qu'une boisson (alcoolisée ou non, ou un mélange de boissons alcoolisées et non alcoolisées). Une boisson peut être tout type de breuvage pouvant être consommé, par exemple : des jus (jus d'orange, multi-fruits, etc.), colas, boissons énergisantes (boissons à base de caféine et de taurine), limonades, des boissons alcoolisées de type vins (rouge, blanc, rosés, pétillants ou non, créments, champagnes), bières, alcools forts (whiskey, vodka, gin, téquila, etc.), ou cocktails à base d'alcools forts et de boissons non alcoolisées (jus, limonades, cola, boissons énergisantes, etc.).

L'électrode de travail Ew est composée d'un métal noble tel que le platine, l'iridium, le palladium, l'or, ou un alliage de ces métaux. De préférence, l'électrode choisie sera en platine. Avantageusement, le platine permet de générer un courant plus élevé que les autres matériaux tout en ayant un coût moins élevé. L'électrode de travail Ew peut se trouver sous des formes et dimensions très variées : fil, disque, broche, couche mince déposée sur un autre métal ou collecteur de charges, etc. La surface de l'électrode de travail Ew peut être comprise entre typiquement 0.002 mm² et 100 mm². La contre-électrode (ou électrode auxiliaire) peut se présenter sous la forme de tout métal résistant aux conditions d'utilisation d'un contenant comprenant les électrodes (par exemple produits de lave-vaisselle, boissons acides corrosives, etc.), comme par exemple les mêmes métaux que l'électrode de travail Ew, ou encore un inox. De préférence, la contre-électrode est en platine. La surface de la contre-électrode Ec est de préférence au moins aussi grande que celle de l'électrode de travail Ew. De manière générale, l'électrode de référence Eref possède un potentiel qui reste pratiquement constant dans les conditions d'une mesure électrochimique et sert de référence pour déterminer le potentiel appliqué à l'électrode de travail Ew. L'électrode de référence Eref peut être sous la forme Ag|AgCl, électrode Calomel, etc. Cependant, ces dernières sont difficiles à mettre en oeuvre pour les applications visées (coût élevé, problème de stabilité à long terme dans les boissons, fragilités dans les conditions d'usage, non-compatible lave-vaisselle, etc.). Ainsi, de préférence, l'électrode de référence Eref peut être une pseudo-électrode de référence. Pour cela, le platine offre de bonnes performances, comme la plupart des métaux de la famille des platinoïdes. Puisque cette électrode n'est présente que pour fournir un potentiel de référence, sa taille n'est pas critique.

Les électrodes Ew, Ec, Eref peuvent par exemple faire partie d'un capteur Capt qui lui-même fait partie d'un contenant (tel qu'un verre) dans lequel la solution Sol est placée.

Pour réaliser les mesures, les trois électrodes Ew, Ec, Eref doivent être positionnées dans la solution Sol (par exemple la boisson). Ainsi quand il s'agit d'un verre, un positionnement en fond de verre sera privilégié. L'espacement entre les électrodes n'est pas critique. On privilégiera quand même de rapprocher au maximum les électrodes afin de faciliter le passage du courant dans le circuit. En effet, si la conductivité ionique de la solution est faible, il sera plus difficile pour le courant de circuler dans le circuit si les électrodes sont éloignées. Dans un exemple, les électrodes sont rapprochées au centre du contenant Cont les comprenant. Ainsi, les électrodes peuvent être immergées, même lorsque que le contenant est incliné (quand un utilisateur du contenant boit par exemple).

Au bloc E1, le procédé comprend l'étape d'alterner, au moyen du circuit potentiostatique CP et pendant une durée prédéterminée, une tension de polarisation de l'électrode de travail entre une tension minimum et une tension maximum prédéterminées, la tension minimum étant choisie dans une gamme de potentiels produisant un courant de réduction du gamma-hydroxybutyrate, la tension maximum étant choisie à l'extérieur de ladite gamme de potentiels. L'étape d'alternance entre deux tensions prédéterminées permet de détecter le GHB dans un verre en mouvement, c'est-à-dire lorsque qu'il est utilisé par un utilisateur. De plus, l'alternance permet la détection du GHB même lorsque la boisson ne contient pas d'alcool.

En particulier, la tension de polarisation de l'électrode de travail Ew est alternée entre deux potentiels distincts, l'un dans la gamme de potentiels produisant un courant de réduction du GHB, et l'autre dans une gamme de potentiels inférieurs (plus proche de zéro). Par exemple, la tension de polarisation de l'électrode de travail Ew peut être variée de manière répétée dans le temps entre une tension maximale comprise dans la gamme -1.4 V/Pt à 0 et une tension minimum comprise dans la gamme -1.6 V/Pt à -2.2 V/Pt, l'électrode de référence étant en platine. La gamme de tension de polarisation peut dépendre du pH de la solution Sol. En effet, le potentiel de référence varie avec le pH de la solution Sol. Il est noté que la notation V/Pt signifie que la tension appliquée à l'électrode de travail, exprimée en volts (V), est relative au potentiel d'une électrode de référence en platine. De façon générale, le potentiel est appliqué par rapport à un potentiel de référence, qui est donné par l'électrode de référence. Dans le cas présent, cela signifie que l'électrode de référence utilisée est en platine (plus précisément, un fil de platine).

Concernant la valeur de tension minimum, en dessous de -1.6 V/Pt, la valeur de la tension peut être trop faible (en valeur absolue) et ainsi la réduction du GHB n'a pas lieu et sa détection ne peut donc pas se produire. Au-dessus de -2.2 V/Pt, la tension est proche de la valeur à partir de laquelle l'eau commence à s'électrolyser et l'électrode vient alors réduire l'eau présente dans la solution Sol. Cela provoque une production de bulles sur l'électrode, qui engendre à son tour un signal bruité. Concernant la tension maximum, l'application d'une tension entre 0 et -1.4 V/Pt a peu d'influence sur la réponse. Au-dessus de -1.4 V, le GHB commence à être réduit et donc la différence de courant produit pour les deux valeurs de tension, 0V/Pt et -1.4V/Pt, diminue. Par conséquent, la sensibilité de la mesure diminue.

La tension potentiostatique est alternée entre la tension minimum et la tension maximum qui ont chacune une durée d'impulsion prédéterminée. Par exemple, la durée d'impulsion de la tension minimum et la tension maximum peut être comprise entre 1 s et 5 s. En effet, cette gamme de durée d'impulsion permet d'optimiser la sensibilité du capteur tout en optimisant le temps de réponse.

Au bloc E2, le procédé P comprend l'étape de mesurer, au moyen du circuit potentiostatique, un courant au niveau de l'électrode de travail Ew pendant ladite durée prédéterminée.

Par exemple, le courant en continu est mesuré pendant la durée prédéterminée.

Au bloc E3, le procédé P comprend l'étape d'analyser le courant mesuré, une détection d'une variation du courant entre un premier instant et un deuxième instant ultérieur au premier instant au cours de ladite analyse étant caractéristique de l'introduction de gamma-hydroxybutyrate.

Le courant présentant une variation en présence du GHB est un courant de réduction du GHB.

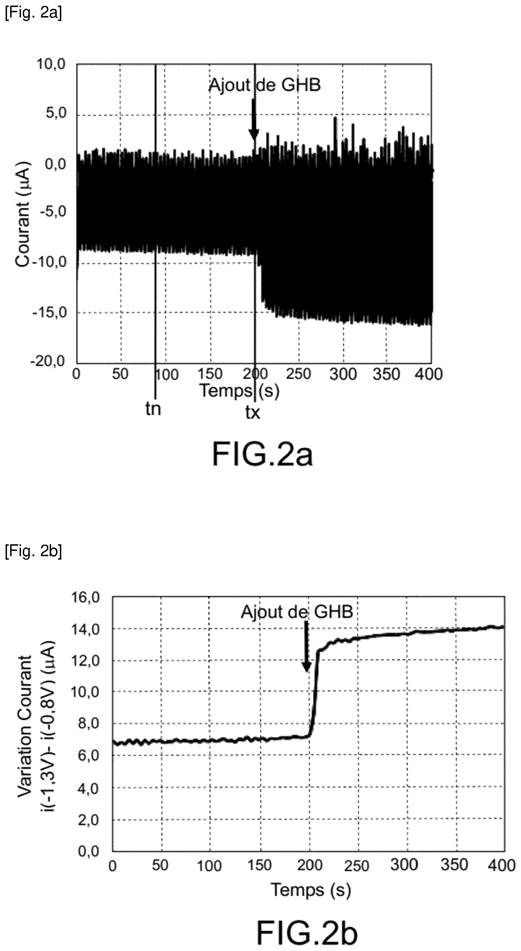

Afin d'illustrer le procédé P, un exemple est présenté dans les figures 2a-2b.

Dans cet exemple, les électrodes Ew, Eref, Ec sont immergées dans un verre comportant un mélange de 70 % d'une boisson énergisante et 30 % de vodka comportant un taux d'alcool de 40%vol. La boisson est agitée en permanence à l'aide d'un barreau magnétique. La tension de polarisation de l'électrode de travail Ew est alternée toutes les 2 secondes entre -0.8 V/Pt et -1.6 V/Pt. A l'instant tx = 200 s, 1 g de GHB pour 200 mL de boisson est ajouté au verre.

Le courant résultant est mesuré au niveau de l'électrode de travail Ew et la figure 2a est obtenue. La partie haute de la courbe de la figure 2a représente le courant mesuré lorsque que la tension potentiostatique est maximum et la partie basse de la courbe représente la tension potentiostatique est minimum.

Comme représenté sur la figure 2a, un courant est observé lors de l'ajout de GHB à tx=200 s comparé par exemple à l'instant tn, l'instant tn étant antérieur à l'instant tx.

Dans un exemple, l'introduction du gamma-hydroxybutyrate est détectée lorsque la variation du courant est supérieure ou égale à une valeur prédéfinie. Par exemple, la valeur prédéfinie peut être un pourcentage de variation, sur la courbe. Par exemple, si le courant varie de plus de 2%, l'introduction du gamma-hydroxybutyrate est détectée. Dans un autre exemple, un seuil peut être défini en fonction des électrodes utilisées, de la solution utilisée et de la tension potentiostatique utilisée.

Par exemple, dans l'exemple de la figure 2a, un seuil peut être prédéfini et lorsque que le courant est inférieur au seuil prédéfini, cela signifie que du GHB a été introduit dans la solution Sol.

Dans un exemple, l'étape E3 d'analyser le courant mesuré peut comprendre la sous-étape de déterminer une valeur moyenne du courant correspondant à une différence entre une valeur moyenne du courant lorsque la tension de polarisation de l'électrode de travail Ew est égale à la tension minimum et une valeur moyenne du courant lorsque la tension de polarisation de l'électrode de travail Ew est égale à la tension maximum en fonction du temps. La variation de la valeur moyenne du courant entre un premier instant et un deuxième instant ultérieur au premier instant au cours de ladite analyse étant caractéristique de l'introduction de gamma-hydroxybutyrate.

Comme illustré dans la figure 2b, une autre façon de visualiser les données est de soustraire la valeur moyenne de courant mesurée pendant une polarisation à -1.6 V/Pt à la valeur moyenne de courant mesuré pendant la polarisation suivante à -0.8 V/Pt pendant la durée de l'expérience. La figure 2b est alors obtenue, dans laquelle une variation d'amplitude très significative est effectivement observée entre les instants tn avant l'ajout du GHB et à l'instant tx lors de l'ajout de GHB à l'instant tx, et ce malgré l'agitation de la boisson.

Dans un autre exemple, l'étape E3 d'analyser le courant mesuré peut également comprendre la sous-étape de déterminer une première différence entre la valeur moyenne du courant au premier instant et la valeur moyenne du courant au deuxième instant et détecter l'introduction du gamma-hydroxybutyrate lorsque la valeur absolue de la première différence est supérieure à un premier seuil prédéterminé.

Ainsi, lorsque la valeur moyenne est déterminée, une différence entre la valeur moyenne du courant à l'instant tx et l'instant tn peut être calculée. Si cette différence est supérieure à un seuil qui a été prédéterminé en fonction des électrodes utilisées, de la solution utilisée et de la tension potentiostatique utilisée, cela indique qu'il y a présence de GHB.

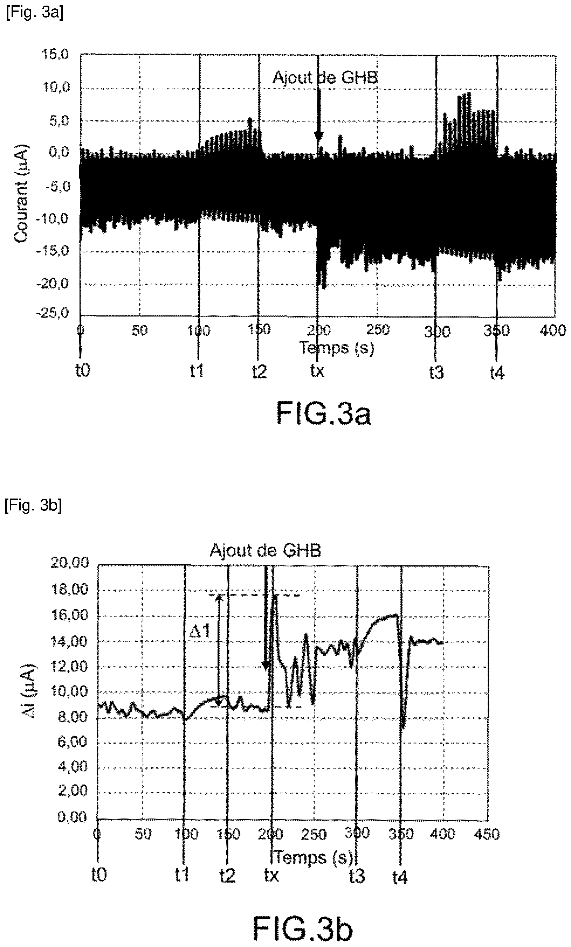

Afin d'illustrer le procédé P, un autre exemple est présenté dans les figures 3a-3d.

Dans cet exemple, les électrodes Ew, Eref, Ec sont immergées dans un verre comportant un mélange de 70 % d'une boisson énergisante et 30% de vodka comportant un taux d'alcool de 40%vol. La tension de polarisation de l'électrode de travail est alternée toutes les 2 secondes entre -0.8 V/Pt et -1.6 V/Pt. Le verre est pris en main sur la période de t0 à t1 = 0 - 100s. Lorsque le verre est pris en main, cela entraine une légère agitation de la boisson. Le verre est ensuite posé sur une table entre t1 = 100 s et t2 = 150 s, puis repris en main entre t2 = 150 s et t3 = 300 s et reposé sur la table à t3 = 300 s et enfin repris en main à t4 = 350 s jusqu'à la fin de l'expérience. A tx = 200 s, l'équivalent de 1 g de GHB dans 200 mL de la boisson est ajouté au verre.

Le courant est mesuré et représenté dans la figure 3a. Lorsque le GHB est introduit à tx = 200 s, une variation du courant est observée dû à la réaction de réduction lors de l'introduction du GHB.

Cependant, il peut être observé que l'agitation du verre (verre au repos sur la table et la prise en main du verre) entraine également des variations du courant mesuré. En effet, une augmentation du courant est observée lorsque la tension de polarisation est égale à -0.8 V/Ptlorsque le verre est posé sur la table, en présence ou non du GHB (la partie haute de la courbe de la figure 3a représente le courant mesuré lorsque que la tension potentiostatique est maximum (ici -0.8 V/Pt) et la partie basse de la courbe représente le courant lorsque la tension potentiostatique est minimum (ici -1.6 V/Pt).

Ainsi, comme dans l'exemple de la figure 2b, la recherche d'une augmentation du courant correspondant à la soustraction de la valeur moyenne de courant mesuré pendant une polarisation à -1.6 V/Pt à la valeur moyenne de courant mesuré pendant la polarisation suivante à -0.8 V/Pt peut conduire à des faux positifs. En effet, l'amplitude augmente également lorsque le verre est posé sur la table, en présence ou non de GHB.

Par ailleurs, lorsque la tension de polarisation est égale à -1.6 V/Pt en présence de GHB, une diminution du courant apparait, comme observé dans l'exemple précèdent de la figure 2a. L'évolution du courant lorsque la tension de polarisation est égale à -1.6 V/Pt en présence de GHB semble relativement insensible au fait que le verre soit agité ou non. Ainsi, la partie basse de la courbe représentant le courant lorsque la tension potentiostatique est minimum peut être utilisée seule afin de déterminer si du GHB a été introduit.

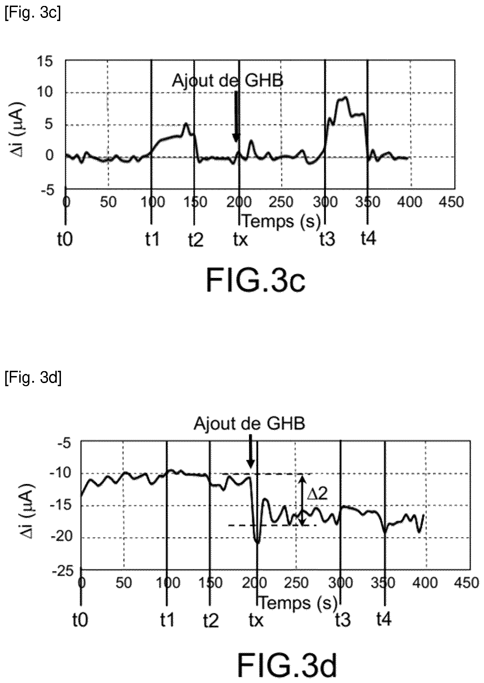

Ainsi, dans l'exemple de la figure 3d, la partie basse de la courbe de la figure 3a est analysée. Afin d'obtenir la figure 3d, l'étape d'analyse E3 comprends les sous-étapes suivantes :

Une sous-étape consiste à déterminer une valeur moyenne du courant lorsque la tension de polarisation de l'électrode de travail est égale à la tension minimum en fonction du temps. Par exemple, la valeur moyenne du courant peut être mesurée pendant une durée prédéterminée. En particulier, la partie basse de la courbe peut être lissée en faisant la moyenne de plusieurs points lorsque la tension de polarisation est égale à -1.6 V. Par exemple une moyenne de 10 points peut être faite. Par la suite, un point désigne une valeur prise sur la courbe en un point de ladite courbe.

Une sous-étape consiste à déterminer une deuxième différence Δ2 entre la valeur moyenne du courant au premier instant et la valeur moyenne du courant au deuxième instant en fonction du temps. Par exemple, la deuxième différence Δ2 est déterminée entre les instants tx et t0.

Une sous-étape consiste à détecter l'introduction du gamma-hydroxybutyrate lorsque la valeur absolue de la deuxième différence Δ2 est supérieure à un deuxième seuil prédéterminé. Par exemple, la valeur prédéfinie peut être le deuxième seuil prédéterminé.

Ainsi, sur la figure 3d, il est observé que la valeur moyenne du courant reste inchangée même lorsque le verre est pris en main ou lorsque qu'il est posé sur la table. Cependant, une variation est bien observée lorsque le GHB est introduit. Cette variation peut être caractérisée en déterminant la différence Δ2 entre deux valeurs moyennes de courant à deux instants différents, la différence Δ2 étant comparée à un seuil qui peut être la valeur prédéfinie.

Dans un autre exemple, tel que représenté sur la figure 3b, l'étape d'analyser peut comprendre les sous-étapes suivantes :

Tel qu'également représenté dans l'exemple de la figure 2b, une sous étape peut comprendre déterminer une valeur moyenne du courant correspondant à une différence entre une valeur moyenne du courant lorsque la tension de polarisation de l'électrode de travail est égale à la tension minimum et une valeur moyenne du courant lorsque la tension de polarisation de l'électrode de travail est égale à la tension maximum en fonction du temps. Par exemple, la partie basse de la courbe peut être lissée en faisant la moyenne de plusieurs points lorsque la tension de polarisation est égale à -1.6 V. Par exemple une moyenne des valeurs de 10 points peut être faite. De même, la partie haute, de la courbe peut être lissée en faisant la moyenne de plusieurs points lorsque la tension de polarisation est égale à -0.8 V. Par exemple une moyenne de 10 points peut être faite. La différence entre les deux moyennes est ensuite faite et tracée en fonction du temps.

Une sous-étape comprend de déterminer une première différence Δ1 entre la valeur moyenne du courant au premier instant et la valeur moyenne du courant au deuxième instant. Par exemple, la différence Δ1 entre la valeur moyenne du courant est déterminée entre les instants tx et t0.

Une sous-étape peut comprendre de détecter l'introduction du gamma-hydroxybutyrate lorsque la valeur absolue de la première différence Δ1 est supérieure à un premier seuil prédéterminé. Par exemple, la valeur prédéfinie peut être le premier seuil prédéterminé. Le premier seuil et le deuxième seuil peuvent être identiques.

Comme indiqué ci-dessus, cette analyse permet de déterminer s'il y a eu une variation du courant. Cependant, les variations du courant peuvent être engendrées par une introduction du GHB ou des vibrations du verre.

Ainsi, afin d'éviter les faux positifs, l'étape d'analyser comprenant les sous-étapes suivantes :
Une sous-étape comprend déterminer une valeur moyenne du courant lorsque la tension de polarisation de l'électrode de travail est égale à la tension maximum en fonction du temps. En particulier, la partie haute de la courbe de la figure 3a représente la tension de polarisation de l'électrode de travail est égale à la tension maximum (dans cet exemple -0.8 V) en fonction du temps. Cette sous-étape consiste à lisser cette partie de la courbe. La moyenne de plusieurs points est calculée lorsque la tension de polarisation est égale à -0.8 V. Par exemple une moyenne de 10 points peut être faite. La courbe obtenue est tracée dans la figure 3c.

Une sous-étape comprend détecter l'introduction du gamma-hydroxybutyrate lorsque la valeur absolue de la première différence est supérieure au premier seuil prédéterminé et la valeur moyenne du courant lorsque la tension de polarisation de l'électrode de travail égale à la tension maximum est environ constante entre le premier instant et le deuxième instant. La tension maximum peut être environ constante lorsqu'elle est en dessous d'un seuil prédéterminé. En d'autres termes, si, pour une même période, la courbe de la figure 3c est environ constante mais que la courbe de la figure 3b présente une première différence qui est supérieure au premier seuil, alors il y a eu une introduction de GHB. Si dans le cas contraire, si, pour une même période, la courbe de la figure 3c présente une variation et la courbe de la figure 3b présente une première différence qui est supérieure au premier seuil, alors du GHB n'a pas été introduit mais le verre a été bougé. Par exemple, la variation de la courbe de la figure 3c peut être une différence entre deux instants comparée à un seuil prédéterminé.

Ainsi, afin d'éviter les faux positifs les étapes d'analyses permettant d'obtenir les figures 3b et 3c peuvent être combinées.

De plus, afin d'obtenir une méthode plus précise, il est possible de combiner les étapes d'analyses permettant d'obtenir les figures 3b, 3c et 3d. Par exemple, la méthode permettant d'obtenir la courbe de la figure 3d permet de déterminer s'il y a une variation représentant une introduction du GHB. Afin de confirmer ce résultat, la méthode permettant d'obtenir la courbe de la figure 3b peut être réalisée afin de confirmer la variation du courant pour une même période de temps. De plus, il est possible de vérifier si la variation obtenue par la méthode permettant d'obtenir la courbe de la figure 3b n'est pas seulement le résultat d'un mouvement du contenant en appliquant la méthode permettant d'obtenir la courbe de la figure 3c.

Il est noté que les seuils décrits ci-dessus et les variations peuvent également être des pourcentages d'augmentation ou de réduction du courant.

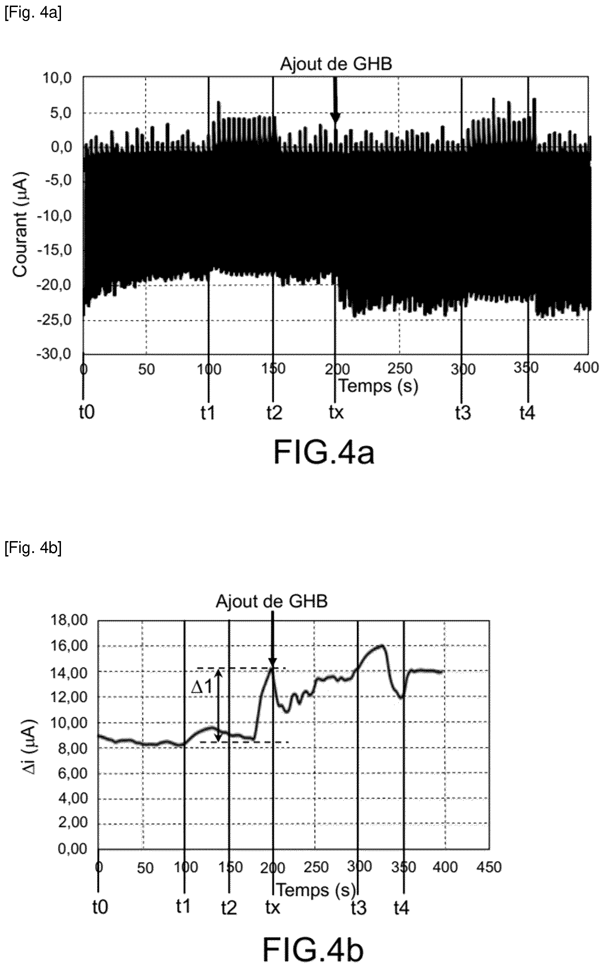

Afin d'illustrer de façon plus approfondie le procédé P, un autre exemple est présenté sur les figures 4a à 4g.

Dans un autre exemple, les électrodes Ew, Eref, Ec sont immergées dans un verre comportant un mélange de 70 % de jus d'orange et 30 % de vodka comportant un taux d'alcool de 40%vol. La tension de polarisation de l'électrode de travail est alternée toutes les 2 secondes entre -0.8 V/Pt et -1.6 V/Pt. Le verre est pris en main sur la période entre t1 = 100 s et t2 = 150s, puis repris en main entre t2 = 300 s et t3 = 350. Le reste du temps, il est posé sur la table. A tx = 200 s, l'équivalent de 1g de GHB dans 200 mL de la boisson est ajouté au verre.

Le courant est mesuré et présenté dans la Figure 4a. Lorsque le GHB est introduit à tx = 200 s, une variation du courant est observée.

Cependant, il peut être observé que l'agitation du verre (verre au repos sur la table et la prise en main du verre) entraine également des variations du courant. En effet, une augmentation du courant est observée lorsque la tension de polarisation est égale à -0.8V lorsque le verre est pris en main, en présence ou non du GHB (la partie haute de la courbe de la figure 4a représente le courant mesuré lorsque que la tension potentiostatique est maximum (ici -0.8 V/Pt) et la partie basse de la courbe représente le courant lorsque la tension potentiostatique est minimum (ici -1.6 V/Pt).

Ainsi, comme dans l'exemple de la figure 2b et 3b, la recherche d'une augmentation du courant correspondant à la soustraction de la valeur moyenne de courant mesurée pendant une polarisation à -1.6 V/Pt à la valeur moyenne de courant mesuré pendant la polarisation suivante à -0.8 V/Pt peut conduire à des faux positifs. En effet, l'amplitude augmente également lorsque le verre est posé sur la table, en présence ou non de GHB.

Par ailleurs, lorsque la tension de polarisation est égale à -1.6 V/Pt en présence de GHB, une diminution du courant apparait, comme observé dans l'exemple précèdent de la figure 2a. L'évolution du courant lorsque la tension de polarisation est égale à -1.6 V/Pt en présence de GHB semble relativement insensible au fait que le verre soit agité ou non. Ainsi, encore une fois, la partie basse de la courbe représentant le courant lorsque la tension potentiostatique est minimum peut être utilisée seule afin de déterminer si du GHB a été introduit.

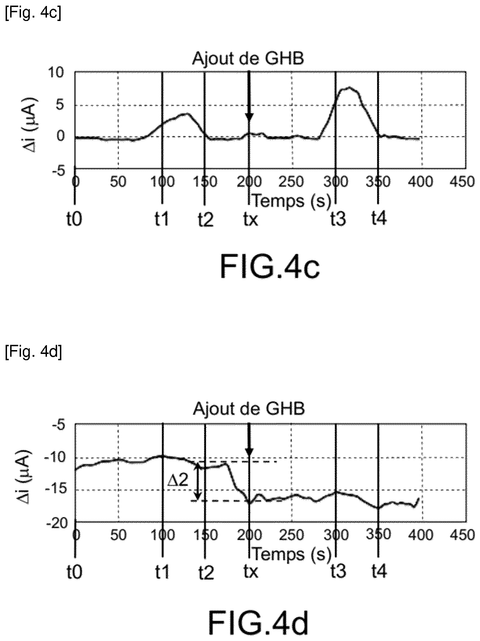

Ainsi, dans l'exemple de la figure 4d, la partie basse de la courbe de la figure 3a est analysée. Afin d'obtenir la figure 4d, l'étape d'analyse E3 comprends les sous-étapes suivantes :

Une sous-étape consiste à déterminer une valeur moyenne du courant lorsque la tension de polarisation de l'électrode de travail est égale à la tension minimum en fonction du temps. Par exemple, la valeur moyenne du courant peut être mesurée pendant une durée prédéterminée. En particulier, la partie basse de la courbe peut être lissée en faisant la moyenne de plusieurs points lorsque la tension de polarisation est égale à -1.6 V. Par exemple une moyenne de 10 points peut être faite.

Une sous-étape consiste à déterminer une deuxième différence Δ2 entre la valeur moyenne du courant au premier instant et la valeur moyenne du courant au deuxième instant en fonction du temps. Par exemple, la deuxième différence Δ2 est déterminée entre les instants t0 et tx.

Une sous-étape consiste à détecter l'introduction du gamma-hydroxybutyrate lorsque la valeur absolue de la deuxième différence Δ2 est supérieure à un deuxième seuil prédéterminé. Par exemple, le deuxième seuil prédéterminé peut être la valeur prédéfinie.

Ainsi, dans la figure 4d, il est observé que la valeur moyenne du courant reste inchangée même lorsque le verre est pris en main ou lorsque qu'il est posé sur la table. Cependant, une variation est bien observée lorsque le GHB est introduit. Cette variation peut être caractérisée en déterminant la deuxième différence Δ2 entre deux valeurs moyennes de courant à deux instants différents, la deuxième différence Δ2 étant comparée à un seuil qui peut être la valeur prédéfinie.

Dans un autre exemple, tel qu'observé dans la figure 4b, l'étape d'analyser peut comprendre les sous-étapes suivantes :

Tel qu'également représenté dans l'exemple des figures 2b et 3b, une sous étape peut comprendre déterminer une valeur moyenne du courant correspondant à une différence entre une valeur moyenne du courant lorsque la tension de polarisation de l'électrode de travail est égale à la tension minimum et une valeur moyenne du courant lorsque la tension de polarisation de l'électrode de travail est égale à la tension maximum en fonction du temps. Par exemple, la partie basse de la courbe peut être lissée en faisant la moyenne de plusieurs points lorsque la tension de polarisation est égale à -1.6 V. Par exemple, une moyenne de 10 points peut être faite. De même, la partie haute, de la courbe peut être lissée en faisant la moyenne de plusieurs points lorsque la tension de polarisation est égale à -0.8 V. Par exemple, une moyenne de 10 points peut être faite. La différence entre les deux moyennes est ensuite faite et tracée en fonction du temps.

Une sous-étape comprend déterminer une première différence Δ1 entre la valeur moyenne du courant au premier instant et la valeur moyenne du courant au deuxième instant. Par exemple, la première différence Δ1 entre la valeur moyenne du courant est déterminée entre les instants t0 ettx.

Une sous-étape peut comprendre détecter l'introduction du gamma-hydroxybutyrate lorsque la valeur absolue de la première différence Δ1 est supérieure à un premier seuil prédéterminé. Par exemple, la valeur prédéfinie peut être le premier seuil prédéterminé. Le premier seuil et le deuxième seuil peuvent être identiques.

Comme indiqué ci-dessus, cette analyse permet de déterminer s'il y a eu une variation du courant. Cependant, les variations du courant peuvent être engendrées par une introduction du GHB ou des vibrations du verre.

Ainsi, afin d'éviter les faux positifs, l'étape d'analyser comprenant les sous-étapes suivantes :

Une sous-étape comprend déterminer une valeur moyenne du courant lorsque la tension de polarisation de l'électrode de travail est égale à la tension maximum en fonction du temps. En particulier, la partie haute de la courbe de la figure 4a représente la tension de polarisation de l'électrode de travail étant égale à la tension maximum (dans cet exemple - 0.8 V) en fonction du temps. Cette sous-étape consiste à lisser la partie haute de la courbe. La moyenne de plusieurs points est calculée lorsque la tension de polarisation est égale à - 0.8 V. Par exemple une moyenne de 10 points peut être faite. La courbe obtenue est tracée dans la figure 4c.

Une sous-étape comprend détecter l'introduction du gamma-hydroxybutyrate lorsque la valeur absolue de la première différence est supérieure au premier seuil prédéterminé et la valeur moyenne du courant lorsque la tension de polarisation de l'électrode de travail égale à la tension maximum est environ constante entre le premier instant et le deuxième instant. La tension maximum peut être environ constante lorsqu'elle est en dessous d'un seuil prédéterminé. En d'autres termes, si, pour une même période, la courbe de la figure 4c est environ constante mais que la courbe de la figure 4b présente une première différence qui est supérieure au premier seuil, alors il y a eu une introduction de GHB. Dans le cas contraire, si, pour une même période, la courbe de la figure 4c présente une variation et la courbe de la figure 4b présente une première différence qui est supérieure au premier seuil, alors du GHB n'a pas été introduit mais le verre a été bougé. Par exemple, la variation de la courbe de la figure 4c peut être une différence entre deux instants comparée à un seuil prédéterminé.

Comme indiqué précédemment, afin d'éviter les faux positifs les étapes d'analyses permettant d'obtenir les figures 4b et 4c ou 4b, 4c et 4d peuvent être combinées.

Dans un mode de réalisation, le procédé comprend l'étape suivante : si le courant présente une variation, émettre un signal d'alerte. Ainsi, l'utilisateur peut être informé d'une introduction de GHB dans sa boisson. Par exemple, le signal d'alerte peut être émis lorsque la variation est supérieure à la valeur prédéfinie pendant une deuxième durée prédéterminée. Par exemple, le signal d'alerte peut être émis lorsque la variation est supérieure à la valeur prédéfinie pendant 10 s ou plus. Le signal d'alerte peut être un signal sonore, lumineux ou vibrant. Par exemple, lorsqu'il est détecté que du GHB est introduit dans le verre d'un utilisateur, un signal vibrant peut être émis afin d'informer l'utilisateur que du GHB a été introduit dans son verre.

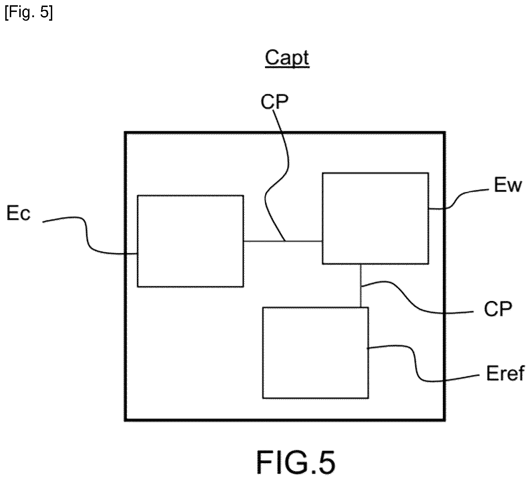

L'invention concerne également un capteur de détection d'introduction de gamma-hydroxybutyrate dans une solution. La figure 5 illustre un exemple de capteur selon l'invention.

Le capteur Capt de détection d'introduction de gamma-hydroxybutyrate dans une solution Sol le capteur est placé dans un contenant Cont et est en contact avec la solution Sol. Le capteur comprend :
- une électrode de travail Ew, une électrode de référence Eref et une contre électrode Ec ;
- un circuit potentiostatique CP, connecté à l'électrode de travail Ew, à l'électrode de référence Eref et à la contre-électrode Ec configuré pour alterner, pendant une durée prédéterminée, une tension de polarisation de l'électrode de travail entre une tension minimum et une tension maximum prédéterminées, la tension minimum étant choisie dans une gamme de potentiels produisant un courant de réduction du gamma-hydroxybutyrate, la tension maximum étant choisie à l'extérieur de la gamme de potentiels, et mesurer, un courant au niveau de l'électrode de travail pendant ladite durée prédéterminée ;
- une unité de traitement et d'analyse de données des moyens de traitements et d'analyse de données configurée pour analyser le courant mesuré, une détection d'une variation du courant entre un premier instant et un deuxième instant ultérieur au premier instant au cours de ladite analyse étant caractéristique de l'introduction de gamma-hydroxybutyrate.

Le capteur Capt est configuré pour mettre en oeuvre le procédé P décrit ci-dessus.

Dans un mode de réalisation, l'électrode de travail Ew est en métal noble, de préférence en platine. L'utilisation d'un métal noble permet ainsi au capteur de résister aux conditions d'utilisation tels que le lavage en lave-vaisselle, les produits ménagers, les boissons acides etc. De plus, le capteur est réutilisable. Dans un exemple, les trois électrodes Ew, Ec, Eref sont en platine.

Dans un mode de réalisation, l'électrode de travail a une surface comprise entre 0.002 mm² et 100 mm². En dessous de 0.002 mm², l'électrode de travail est une microélectrode. Le courant mesuré des microélectrodes est faible et nécessite donc une électronique plus coûteuse (comme l'utilisation d'amplificateurs). De plus, dans le cas des boissons gazeuses, l'accrochage d'une bulle sur une microélectrode risquerait de perturber le signal de mesure de façon significative. Cependant, le platine étant un matériau coûteux, il peut être intéressant de limiter la taille de l'électrode à 100 mm², par exemple.

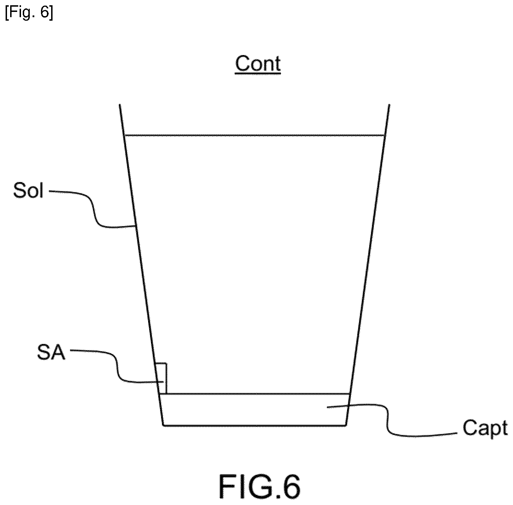

L'invention concerne également un contenant Cont permettant la détection d'une introduction de gamma-hydroxybutyrate dans une solution Sol. La figure 6 illustre un exemple de contenant Cont selon l'invention.

Le contenant Cont permettant la détection d'une introduction de gamma-hydroxybutyrate dans la solution Sol placée dans le contenant Cont, comprend le capteur Capt décrit ci-dessus.

Dans un mode de réalisation, le contenant Cont comprend un système d'alerte SA configuré pour émettre le signal d'alerte, le signal d'alerte étant de préférence un signal sonore, lumineux ou vibrant. Dans un autre mode de réalisation, le capteur Capt comprend le système d'alerte SA.

L'application concerne l'intégration d'un capteur Capt mettant en oeuvre le procédé P de l'invention décrit ci-dessus, qui serait intégré à un verre de boisson pour permettre au consommateur d'être informé au plus vite de l'introduction de GHB dans son verre. Elle vise principalement le secteur évènementiel (boites de nuits, traiteurs, milieux festifs en général, événements sportifs, etc.).

Bien que l'invention ait été illustrée et décrite en détail à l'aide d'un mode de réalisation préféré, l'invention n'est pas limitée aux exemples divulgués. D'autres variantes peuvent être déduites par l'homme du métier sans sortir du cadre de protection de l'invention revendiquée. Par exemple, les exemples de boissons utilisées ne servent qu'à illustrer l'application.

## Revendications

1. Procédé de détection (P) d'introduction de gamma-hydroxybutyrate dans une solution (Sol) au moyen d'un capteur (Capt), le capteur comprenant une électrode de travail (Ew), une électrode de référence (Eref), une contre-électrode (Ec) et un circuit potentiostatique (CP) connecté à l'électrode de travail (Ew), l'électrode de référence (Eref) et la contre-électrode (Ec), le procédé comprenant les étapes suivantes :
- alterner, au moyen du circuit potentiostatique et pendant une durée prédéterminée, une tension de polarisation de l'électrode de travail entre une tension minimum et une tension maximum prédéterminées, la tension minimum étant choisie dans une gamme de potentiels produisant un courant de réduction du gamma-hydroxybutyrate, la tension maximum étant choisie à l'extérieur de ladite gamme de potentiels ;
- mesurer, au moyen du circuit potentiostatique, un courant au niveau de l'électrode de travail pendant ladite durée prédéterminée ; et
- analyser le courant mesuré, une détection d'une variation du courant entre un premier instant et un deuxième instant ultérieur au premier instant au cours de ladite analyse étant caractéristique de l'introduction de gamma-hydroxybutyrate.

2. Procédé de détection selon la revendication 1, dans lequel l'introduction du gamma-hydroxybutyrate est détectée lorsque la variation du courant est supérieure ou égale à une valeur prédéfinie.

3. Procédé de détection selon l'une des revendications précédentes, l'étape d'analyse du courant mesuré comprenant les sous-étapes suivantes :
- déterminer une valeur moyenne du courant correspondant à une différence entre une valeur moyenne du courant lorsque la tension de polarisation de l'électrode de travail est égale à la tension minimum et une valeur moyenne du courant lorsque la tension de polarisation de l'électrode de travail est égale à la tension maximum en fonction du temps ;
- déterminer une première différence entre la valeur moyenne du courant au premier instant et la valeur moyenne du courant au deuxième instant; et
- détecter l'introduction du gamma-hydroxybutyrate lorsque la valeur absolue de la première différence est supérieure à un premier seuil prédéterminé.

4. Procédé de détection selon la revendication 3, l'étape d'analyse du courant mesuré comprenant les sous-étapes suivantes :
- déterminer une valeur moyenne du courant lorsque la tension de polarisation de l'électrode de travail est égale à la tension maximum en fonction du temps ;
- détecter l'introduction du gamma-hydroxybutyrate lorsque la valeur absolue de la première différence est supérieure au premier seuil prédéterminé et la valeur moyenne du courant lorsque la tension de polarisation de l'électrode de travail égale à la tension maximum est environ constante entre le premier instant et le deuxième instant.

5. Procédé de détection selon l'une des revendications précédentes, l'étape d'analyse du courant mesuré comprenant les sous-étapes suivantes :
- déterminer une valeur moyenne du courant lorsque la tension de polarisation de l'électrode de travail est égale à la tension minimum en fonction du temps ;
- déterminer une deuxième différence entre la valeur moyenne du courant au premier instant et la valeur moyenne du courant au deuxième instant temps ; et
- détecter l'introduction du gamma-hydroxybutyrate lorsque la valeur absolue de la deuxième différence est supérieure à un deuxième seuil prédéterminé.

6. Procédé de détection selon l'une des revendications précédentes, le procédé comprenant :
- si le courant présente une variation, émettre un signal d'alerte.

7. Procédé de détection selon la revendication 6, dans lequel le signal d'alerte est émis lorsque la variation est supérieure à la valeur prédéfinie pendant une deuxième durée prédéterminée.

8. Procédé de détection selon l'une des revendications 6 ou 7, dans lequel le signal d'alerte est un signal sonore, lumineux ou vibrant.

9. Procédé de détection selon l'une des revendications précédentes, dans lequel la gamme de tension de l'électrode de travail est choisie entre -2.2 V/Pt et -1.6 V/Pt pour la tension minimum et -1.4 V/Pt et 0 V/Pt pour la tension maximum, l'électrode de référence étant en platine.

10. Capteur (Capt) de détection d'introduction de gamma-hydroxybutyrate dans une solution(Sol), le capteur étant placé dans un contenant (Cont) et étant en contact avec la solution, le capteur comprenant une électrode de travail (Ew), une électrode de référence (Eref) et une contre électrode (Ec) et un circuit potentiostatique (CP) connecté à l'électrode de travail (Ew), à l'électrode de référence (Eref) et à la contre-électrode (Ec), le capteur étant configuré pour mettre en oeuvre le procédé selon l'une des revendications 1 à 5.

11. Capteur (Capt) de détection selon la revendication 10, l'électrode de travail étant en métal noble, de préférence en platine.

12. Capteur (Capt) de détection selon la revendication 10 ou la revendication 11, l'électrode de travail ayant une surface comprise entre 0.002 mm² et 100 mm².

13. Contenant (Cont) permettant la détection d'une introduction de gamma-hydroxybutyrate dans une solution (Sol) placé dans le contenant (Cont), le contenant comprenant le capteur des revendications 10 à 12.

14. Contenant selon la revendication précédente, le contenant comprenant un système d'alerte (SA) configuré pour émettre le signal d'alerte, le signal d'alerte étant de préférence un signal sonore, lumineux ou vibrant.
